Europäisches Patentamt

European Patent Office

Office européen des brevets

(19)

(11) Numéro de publication: **0 331 549**
**A1**

## DEMANDE DE BREVET EUROPEEN

(12)

(21) Numéro de dépôt: 89400417.5

(22) Date de dépôt: 15.02.89

(51) Int. Cl.⁴: **C 07 C 25/18**
C 07 C 17/12

(30) Priorité: 02.03.88 FR 8802608

(43) Date de publication de la demande:
06.09.89 Bulletin 89/36

(84) Etats contractants désignés:
AT BE CH DE ES FR GB GR IT LI LU NL SE

(71) Demandeur: **RHONE-POULENC CHIMIE**
**25, quai Paul Doumer**
**F-92408 Courbevoie Cédex (FR)**

(72) Inventeur: **Desmurs, Jean-Roger**
**La Jonquière Route de Ternay**
**Communay F-69360 Saint Symphorien D'Ozon (FR)**

**Nonn, Alain**
**1, Allée de la Gravière**
**F-69110 Sainte Foy les Lyon (FR)**

(74) Mandataire: **Dubruc, Philippe et al**
**RHONE-POULENC CHIMIE Service Brevets Chimie 25,**
**quai Paul-Doumer**
**F-92408 Courbevoie Cédex (FR)**

(54) **Procédé de préparation du dibromo-4,4' biphényle en milieu solvant et avec catalyseur.**

(57) L'invention concerne la préparation du dibromo-4,4' biphényle.

Selon l'invention on fait réagir le biphényle avec du brome dans un solvant et en présence d'un système catalytique à base d'au moins un composant choisi dans le groupe comprenant les acides de Lewis et l'iode.

EP 0 331 549 A1

**Description**

**PROCEDE DE PREPARATION DU DIBROMO-4,4′ BIPHENYLE EN MILIEU SOLVANT ET AVEC CATALYSEUR**

La présente invention concerne la préparation du dibromo-4,4′ biphényle.

Le dibromo-4,4′ biphényle est un composé donnant accès par hydrolyse au dihydroxy-4,4′ biphényle ce dernier produit étant un monomère pour polymères thermotropes notamment, ou pouvant être utilisé comme stabilisant.

Il existe déjà plusieurs procédés de préparation du dibromo-4,4′ biphényle. Toutefois, le besoin s'est fait sentir d'un procédé industriel permettant d'activer la réaction de bromation et de maintenir conjointement la sélectivité en dibromo-4,4′ biphényle à un niveau élevé.

L'objet principal de l'invention est donc la mise au point d'un tel procédé.

Dans ce but, le procédé selon l'invention de préparation du dibromo-4,4′ biphényle est caractérisé en ce qu'on fait réagir le biphényle avec du brome dans un solvant et en présence d'un système catalytique à base d'au moins un composant choisi dans le groupe comprenant les acides de Lewis et l'iode.

Le procédé de l'invention permet, à température ambiante, d'obtenir des rendements supérieurs à 85 %.

D'autres détails et caractéristiques de l'invention seront mieux compris à la lecture de la description et des exemples concrets mais non limitatifs qui vont suivre.

Selon une première caractéristique de l'invention, le procédé est mis en oeuvre dans un solvant.

Généralement, ce solvant est choisi dans le groupe comprenant les solvants hydrocarbures aliphatiques, cycliques, aromatiques, et leurs équivalents halogénés et les solvants nitrés. Il est préférable d'utiliser des solvants inertes vis-à-vis du brome.

On peut utiliser avantageusement les solvants nitrés car ils permettent des temps de réaction beaucoup plus réduits.A titre d'exemple on peut citer le tétrachlorure de carbone, le dichlorométane, le dichloro-1,2-éthane, l'o-dichlorobenzène, le nitrobenzène.

La concentration en biphényle dans le milieu de départ peut être comprise entre 0,1 et 3 moles/l. De préférence elle est comprise entre 1 et 2 moles/l.

La deuxième caractéristique de l'invention réside dans l'utilisation d'un système catalytique.

Ce système catalytique peut être composé soit d'un acide de Lewis, soit d'iode ou encore d'un mélange des deux.

Comme acide de Lewis on utilise plus particulièrement ceux comprenant un élément appartenant aux groupes IIb, IIIa, IVb, Va, Vb, VIa et VIII de la classification périodique. On peut mentionner dans ce cas le fer, le zinc, l'antimoine, le tellurium, le zirconium, le titane, l'aluminium, le gallium, l'hafnium, le nobium, le phosphore, le bore.

Plus précisément, on met en oeuvre les halogénures de ces éléments tels que par exemple $FeCl_3$, $ZnCl_2$, $SbCl_5$, $PBr_3$, $AlCl_3$, $TiCl_4$, $ZrCl_4$.

Selon un autre mode de réalisation de l'invention, il est possible d'utiliser un système catalytique à base au moins d'un acide de Lewis mais comprenant en outre un composant choisi dans le groupe des amines, des phosphines et des sulfures.

On ne sortirait bien entendu pas du cadre de l'invention en se servant d'un système catalytique à base d'iode, d'un acide de Lewis et/ou des composants du type juste mentionné ci-dessus.

Pour les amines, on peut citer plus particulièrement les amines tertiaires.

Pour les phosphines on peut citer les alkylphosphines, les arylphosphines telles que la triphénylphosphine et les alkylarylphosphines.

Pour les sulfures, on peut indiquer les dialkylsulfures, les diarylsulfures tels que le diphénylsulfure et les dialkylarylsulfures.

D'une manière générale, la quantité de catalyseur sera comprise entre 0,1 et 10 % exprimée en mole par rapport au biphényle engagé et plus particulièrement entre 0,5 et 2 %.

Dans le cas d'un système catalytique à base d'un acide de Lewis et d'un autre composant le rapport en mole composant/acide de Lewis est fonction de la nature du couple composant/acide de Lewis. En général, ce rapport est choisi voisin de 1 ou égal à 1.

La réaction se fait généralement en coulant le brome dans un mélange comprenant le biphényle, le solvant et le système catalytique.

On utilise habituellement un excès de brome compris entre 0 et 20 % par rapport à la stoechiométrie.

La réaction est conduite à une température qui peut varier le plus souvent entre 0 et 60°C.On préfère travailler à température ambiante.

Des exemples de mise en oeuvre du procédé de l'invention vont être donnés ci-dessous.

EXEMPLE 1

Dans un réacteur de 250 ml muni d'une agitation magnétique, d'un réfrigérant, d'une ampoule de coulée et d'un thermomètre, on charge :

15,4 g de biphényle (0,1 mole),
100 ml de solvant,
la quantité d'iode indiquée dans le tableau 1 ci-dessous.

A température ambiante, on coule en 10 minutes le brome (35,2 g - 0,22 mole) puis on agite pendant un temps variable à température ambiante. En fin de réaction, l'excès de brome et l'iode sont détruits par du sulfite ou du bisulfite de sodium.

La phase organique est lavée, séchée et diluée à un volume donné pour être analysée en CPG.

Les autres conditions opératoires et les résultats sont donnés dans le tableau 1 ci-dessous.

TABLEAU 1

| ESSAI | SOLVANT | CATALY-SEUR | DUREE | TAUX DE TRANS-FORMA-TION % | RENDEMENT % | | |
|---|---|---|---|---|---|---|---|
| | | | | | Br-4 a) | diBr-2,4' b) | diBr-4,4' |
| 1A | (CH2Cl)2 | - | 48 H | 100 | 23 | 1 | 46,7 |
| 1B | (CH2Cl)2 | I2(10%) | 30 H | 100 | 2,3 | 2,5 | 65,0 |
| 1C | CH2Cl2 | - | 29 H | 99 | 42 | 1 | 23,6 |
| 1D | CH2Cl2 | I2(10%) | 27 H | 100 | 0 | 2,6 | 64,8 |
| 1E | PhNO2 | I2(10%) | 3H | 100 | 8,2 | 2,5 | 86,2 |

a) cette colonne concerne le rendement en dérive monobromé
b) cette colonne concerne le rendement en dérivé di-bromé mais en positions 2 et 4'.

On voit clairement apparaitre à la lecture du tableau 1 l'intérêt d'un système catalytique du type iode pour la réactivité (rendement en diBr-4,4').

EXEMPLE 2

On utilise le même mode opératoire que dans l'exemple 1 mais en employant comme catalyseur des acides de Lewis.

Les conditions opératoires et les résultats sont donnés dans le tableau 2 ci-dessous.

TABLEAU 2

| ESSAI | SOLVANT | CATALY-SEURS | DUREE | TAUX DE TRANS-FORMA-TION % | RENDEMENT % | | |
|---|---|---|---|---|---|---|---|
| | | | | | Br-4 | diBr-2,4' | diBr-4,4' |
| 2A | CH2Cl2 | FeCl3 (10 %) | 24 H | 100 | 0 | 16,7 | 50,4 + tri |
| 2B | CH2Cl2 | FeCl3 (1 %) | 3H | 100 | 0 | 14,0 | 49,0 |
| 2C | CH2Cl2 | ZnCl2 (1 %) | 29H | 100 | 17,0 | 1,7 | 50,3 |
| 2D | CH2Cl2 | SbCl5 (1%) | 24H | 100 | 9,6 | 2,3 | 56,7 |
| 2E | CH2Cl2 | PBr3 (1 %) | 26H | 100 | 27,0 | 2,1 | 43,6 |

On peut constater à la lecture du tableau 2 l'intérêt d'un acide de lewis pour l'augmentation de la réactivité.

EXEMPLE 3

On utilise toujours le même mode opératoire que précédemment mais avec comme catalyseur un sytème acide de Lewis - iode.

Les autres conditions et les résultats sont donnés dans le tableau 3 ci-dessous.

EP 0 331 549 A1

TABLEAU 3

| ESSAI | SOLVANT | CATALY-SEURS | DUREE | TAUX DE TRANS-FORMA-TION % | RENDEMENT % | | |
|-------|---------|--------------|-------|------------------------------|-------------|---|---|
| | | | | | Br-4 | diBr-2,4' | diBr 4,4' |
| 3A | $CH_2Cl_2$ | $I_2$(1%) $FeCl_3$ (1 %) | 6 H | 100 | 3,3 | 3,4 | 87,4 |

## EXEMPLE 4

Dans un quadricol de 250 ml muni d'un réfrigérant à eau surmonté d'une sortie vers un piège à soude permettant de détruire l'acide bromhydrique formé, d'une ampoule à brome, d'un système d'agitation mécanique et d'un thermomètre, on charge :

30,8 g de biphényle,
100 ml de dichlorométhane,
0,32 g de chlorure ferrique,
0,50 g d'iode.

On ajoute en maintenant le milieu réactionnel à température ambiante par contrôle du débit, 67,8 g de brome goutte à goutte. On maintient à température ambiante pendant 5 heures, puis on chauffe au reflux du dichlorométhane pendant une heure. Après retour à température ambiante, l'excès de brome et l'iode sont détruits par une solution de sulfite de sodium à 10 %.

Le dibromo-4,4'biphényle étant peu soluble dans le dichlorométhane, il peut être filtré et lavé avec ce solvant.

La solution résiduelle est lavée à l'eau jusqu'à neutralité puis séchée sur sulfate de sodium.

Les rendements pour le produit brut obtenus par dosage CPG de la phase organique (mélange cristaux + solution) sont les suivants :

Rendements

| (diBr-4,4') | = | 93,3 % |
| (diBr-2,4') | = | 3,5 % |
| (Br-4) | = | 0,9 %. |

Les exemples 3 et 4 montrent que la combinaison iode-acide de Lewis apporte outre un rendement amélioré une sélectivité élevée (rapport diBr-4,4'/diBr-2,4).

Bien entendu, l'invention n'est nullement limitée aux modes de réalisation décrits qui n'ont été donnés qu'à titre d'exemples. En particulier, elle comprend tous les moyens constituant des équivalents techniques des moyens décrits ainsi que leurs combinaisons si celles-ci sont mises en oeuvre dans le cadre de la protection comme revendiquée.

**Revendications**

1. Procédé de préparation du dibromo-4,4' biphényle caractérisé en ce qu'on fait réagir le biphényle avec du brome dans un solvant et en présence d'un système catalytique à base d'au moins un composant choisi dans le groupe comprenant les acides de Lewis et l'iode.

2. Procédé selon la revendication 1, caractérisé en ce qu'on choisit le solvant dans le groupe comprenant les solvants hydrocarbures aliphatiques, cycliques et aromatiques et leurs équivalents halogénés et les solvants nitrés.

3. Procédé selon la revendication 1 ou 2, caractérisé en ce qu'on choisit les acides de Lewis parmi ceux comprenant un élément appartenant aux groupes IIb, IIIa, IVb, Va, Vb, VIa, et VIII de la classification périodique.

4. Procédé selon la revendication 3, caractérisé en ce qu'on utilise comme acides de Lewis les halogénures des éléments précités.

5. Procédé selon l'une des revendications précédentes, caractérisé en ce qu'on utilise un système catalytique comprenant au moins un acide de Lewis et en outre un composant choisi dans le groupe comprenant les amines, les phosphines, les sulfures.

6. Procédé selon l'une des revendications 1 à 4 caractérisé en ce qu'on utilise un système catalytique comprenant l'iode, et au moins un composant choisi dans le groupe comprenant les acides de Lewis, les amines, les phosphines, les sulfures.

4

7. Procédé selon l'une des revendications précédentes caractérisé en ce qu'on utilise un système catalytique à base d'un acide de Lewis et d'un autre composant et en ce que le rapport en pois composant/acide de Lewis est voisin de 1 ou égal à 1.

8. Procédé selon l'une des revendications précédentes caractérisé en ce que la quantité de catalyseur du système catalytique est comprise entre 0,1 et 10 % exprimée en mole par rapport au biphényle engagé et plus particulièrement entre 0,5 et 2 %.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-3 763 248  (L.C. MITCHELL) <br> * Revendications * <br> --- | 1-4,8 | C 07 C  25/18 <br> C 07 C  17/12 |
| X | CHEMICAL ABSTRACTS, vol. 93, no. 19, 10 novembre 1980, page 621, no. 185926t, Columbus, Ohio, US; & JP-A-80 64 532 (HODOGAYA CHEMICAL CO. LTD) 15-05-1980 <br> * Résumé * <br> --- | 1-2 | |
| X | CHEMICAL ABSTRACTS, vol. 82, no. 3, 20 janvier 1975, page 464, no. 16426x, Columbus, Ohio, US; G.V. MEDOKS et al.: "Bromination of biphenyl", & ISSLED. MAT., FIZ., KHIM. 1973, 124-7 <br> * Résumé * <br> ----- | 1-2 | |

**DOMAINES TECHNIQUES RECHERCHES (Int. Cl.4)**

C 07 C  17/00

Le présent rapport a été établi pour toutes les revendications

| Lieu de la recherche | Date d'achèvement de la recherche | Examinateur |
|---|---|---|
| LA HAYE | 12-05-1989 | VAN GEYT J.J.A. |